# EUROPEAN PATENT SPECIFICATION

(11) **EP 4 130 177 B1**
(45) Date of publication and mention of the grant of the patent: **04.06.2025**
(21) Application number: 21861540.9
(22) Date of filing: 24.08.2021
(51) Int. Cl.: C09G 1/06, C09D 201/04, C09K 3/00, C11D 7/50, C09D 7/20, C10M 105/54, C10M 107/38, C10M 131/10, C10M 147/00

(54) **COMPOSITION CONTAINING FLUORINE OIL**
ZUSAMMENSETZUNG MIT FLUORÖL
COMPOSITION CONTENANT DE L'HUILE FLUORÉE

(30) Priority: 31.08.2020 JP 2020146120
(43) Date of publication of application: 08.02.2023
(60) Divisional application: 24183650.1
(73) Proprietor: Daikin Industries, Ltd., Osaka-shi, Osaka 530-0001 (JP)
(72) Inventor: NAKAUE, Tsubasa, Osaka-shi, Osaka 5300001 (JP); KARUBE, Daisuke, Osaka-shi, Osaka 5300001 (JP); YOSHIYAMA, Asako, Osaka-shi, Osaka 5300001 (JP)
(74) Representative: Hoffmann Eitle
(86) International application number: PCT/JP2021/030945
(87) International publication number: WO 2022/045115

(56) References cited:
- CN-A- 110 317 286
- CN-A- 110 317 286
- CN-A- 112 126 327
- JP-A- 2008 539 312
- JP-A- 2015 143 359
- JP-A- 2019 167 304
- JP-A- 2019 167 304
- JP-A- 2020 132 688
- US-A1- 2015 122 461
- US-A1- 2015 184 049

## Description

### Technical Field

The present disclosure relates to a composition containing a fluorine oil.

### Background Art

Hydrofluoroethers (HFEs) have attracted attention as alternatives to chlorofluorocarbons (CFCs) and hydrochlorofluorocarbons (HCFCs) because of their low global warming potential (GWP), low ozone depletion potential (ODP), and low toxicity.

JP-A-2015-143359 discloses a cleaning agent containing an azeotropic mixture-like composition containing 1,1,1,3,3,3-hexafluoroisopropyl methyl ether (HFE-356mmz), which is an HFE, and hexafluoroisopropanol (HFIP).

JP-A-2011-506681 discloses that an azeotrope-like composition containing 1,1,1,2,3,3-hexafluoro-3-methoxy-propane, which is another HFE, and 1-bromopropane is usable in cleaning processes as a refrigerant.

US-A-2015/184049 discloses a azeotropic mixture-like composition containing HFE-356mmz and HFIP.

JP-A-2019-167304 relates to a method for producing high-purity HFE-356 mmz, comprising contacting a composition containing HFE-356 mmz, HFIP and at least one compound selected from dimethyl ether, halomethane and methyl p-toluenesulfonate with a silica-alumina solid adsorbent.

CN-A-110 317 286 describes method of washing polyvinylidene fluoride resin using a fluorine-containing cleaning agent having a boiling point of above 30°C and being at least one compound selected from chlorofluoroalkanes and (per)fluorinated alkanes, cycloalkanes, ethers, alcohols and ketones.

US-A-2015/122461 concerns a working medium for a boiling-type cooler comprising HFE-356mmz as a main component.

### Summary of Invention

### Technical Problem

An object of the present disclosure is to provide a novel composition containing 1,1,1,3,3,3-hexafluoroisopropyl methyl ether (HFE-356mmz) and/or 1,1,2,3,3,3-hexafluoropropyl methyl ether (HFE-356mec), and a fluorine oil. Another object of the present disclosure is to provide a solvent composition or cleaning composition that contains HFE-356mmz and/or HFE-356mec for removing a fluorine oil.

### Solution to Problem

The present disclosure provides a composition (also referred to as "the present composition" hereinafter) comprising
(A) 1,1,1,3,3,3-hexafluoroisopropyl methyl ether (HFE-356mmz) and/or 1,1,2,3,3,3-hexafluoropropyl methyl ether (HFE-356mec), and
(B) a fluorine oil, which is at least one compound selected from
   - a perfluoropolyether compound,
   - a monocarboxylic acid compound, a dicarboxylic acid compound or a silane compound, each containing a perfluoropolyether group, and
   - a polymer of chlorotrifluoroethylene having a number average molecular weight of ≤ 10,000,
and which composition does not contain hexafluoroisopropanol (HFIP).

Also, the present disclosure provides the use of the present composition in a coating agent which is a mixture existing in liquid form at room temperature and is applied to an article to form a coating film for protecting or imparting luster to the surface of the article.

Furthermore, the present disclosure provides a cleaning method comprising removing a fluorine oil (B) as defined in the present composition above, using a cleaning agent which contains HFE-356mmz and/or HFE-356mec and does not contain HFIP.

Yet further, the present disclosure provides a dissolution method comprising dissolving a fluorine oil (B) as defined in the present composition above, using a solvent which contains HFE-356mmz and/or HFE-356mec and does not contain HFIP.

Preferred embodiments of the invention are as defined in the appended dependent claims and/or in the following detailed description.

### Advantageous Effects of Invention

The present disclosure provides a novel composition containing HFE-356mmz and/or HFE-356mec and a fluorine oil.

### Description of Embodiments

The present disclosure includes the following embodiments.

The present composition contains component (A) and component (B). The component (A) is 1,1,1,3,3,3-hexafluoroisopropyl methyl ether (HFE-356mmz) and/or 1,1,2,3,3,3-hexafluoropropyl methyl ether (HFE-356mec). The component (B) is a fluorine oil.

The fluorine oil, which is component (B), is at least one compound selected from (i) perfluoropolyether compounds, (ii) compounds which contain a perfluoropolyether group and a functional group and which are selected from monocarboxylic acid compounds, dicarboxylic acid compounds and silane compounds, and (iii) polymers of chlorotrifluoroethylene having a number average molecular weight of ≤ 10,000.

Examples of perfluoropolyether compounds include compounds having a constituent unit of the formula -(OCₙF₂ₙ)- (1) wherein n is 1, 2, 3, or 4.

Commercial products of perfluoropolyether compounds include Krytox (registered trademark) GPL oil, 143 oil, vacuum pump oil (trade names, produced by Chemours), Demnum S-20, Demnum S-65, Demnum S-200 (trade names, produced by Daikin Industries, Ltd.), Fomblin M, Fomblin Z, Fomblin Y (trade names, produced by Solvay Specialty Polymers Japan K.K.), and BARRIERTA (trade name, produced by NOK Klüber Co., Ltd.).

Examples of monocarboxylic acid compounds containing a perfluoropolyether group include compounds of formula (2):

Rf²-PFPE²-Z²-COOH

wherein
PFPE² is a group of the formula:
-(OC₆F₁₂)ₐ-(OC₅F₁₀)_{b}-(OC₄F₈)_{c}-(OC₃F₆)_{d}-(OC₂F₄)ₑ-(OCF₂)_{f}-wherein a-f each independently are an integer of 0-200, (a+b+c+d+e+f) is at least 1, and the order of the repeating units in parentheses accompanied by a, b, c, d, e, or f is optional;
Rf² is C₁₋₁₆-alkyl optionally substituted with one or more F; and
Z² is a single bond or a divalent organic group.

Examples of mono- and dicarboxylic acid compounds containing a perfluoropolyether group include compounds of formula (3):

X1-O-Rf-Y

wherein -Rf is a perfluoropolyoxyalkylene chain with a number average molecular weight of 500-10,000 containing a unit of (C₂F₄O), (CF₂O), a (C₃F₆O) type (wherein the (C₃F₆O) unit can be a unit represented by formula -(CF₂CF(CF₃)O) or (CF(CF₃)CF₂O)-), (CF₂(CF₂)zCF₂O)- (wherein z is an integer of 1 or 2), or - CR⁴R⁵CF₂CF₂O- (wherein R⁴ and R⁵ are identical or different and selected from H, Cl, or, for example, C₁₋₄ perfluoroalkyl) with the unit statistically distributed along the main chain; and -X1 and Y is a terminal group and contains at least one carboxyl group, provided that when one terminal group contains only one of these groups, the other terminal group contains a CF₃ group.

Specific examples of dicarboxylic acid compounds containing a perfluoropolyether group include HOOCCF₂-O-[(CF₂CF₂O)₂₈-(CF₂O)₂₂]-CF₂COOH produced by Daikin Industries, Ltd. Specific examples of monocarboxylic acid compounds containing a perfluoropolyether group include CF₃-O-[(CF₂CF₂O)₂₈-(CF₂O)₂₂]-CF₂COOH produced by Daikin Industries, Ltd. Examples of commercial products of silane compounds containing a perfluoropolyether group include KY-100 series produced by Shin-Etsu Chemical Co., Ltd. (e.g., KY-178, KY-185, KY-195), OPTOOL (registered trademark) DSX, OPTOOL (registered trademark) AES, OPTOOL (registered trademark) UF503, and OPTOOL (registered trademark) UD509 produced by Daikin Industries, Ltd., and Afluid (registered trademark) S550 produced by AGC Inc. Specific examples of silane compounds containing a perfluoropolyether group include OPTOOL (registered trademark) UD500 produced by Daikin Industries, Ltd.

Commercial products of polymers of chlorotrifluoroethylene having a number average molecular weight of ≤ 10,000 include DAIFLOIL #1, DAIFLOIL #3, DAIFLOIL #10, DAIFLOIL #20, DAIFLOIL #50, and DAIFLOIL #100 (trade names, produced by Daikin Industries, Ltd.).

In the present disclosure, from the standpoint of achieving sufficient lubricity and sufficient coating film strength, component (B) for use is preferably a fluorine oil with a kinematic viscosity at 20°C of 30-2000 cst or a fluorine oil with a kinematic viscosity at 25°C of 100-2000 cst. In the present disclosure, from the standpoint of further increasing lubricity and coating film strength, component (B) for use is more preferably a fluorine oil with a kinematic viscosity at 20°C of 50-1500 cst or a fluorine oil with a kinematic viscosity at 25°C of 300-1500 cst.

In the present disclosure, preferably, the content of component (A) is 1-99.99 mass%, and the content of component (B) is 0.01-99 mass% based on the total amount of components (A) and (B). When the contents of components (A) and (B) are within these ranges, components (A) and (B) in the composition exhibit excellent solubility, resulting in a uniform composition. In the present specification, the total amount of components (A) and (B) means that the sum of the content of components (A) and (B) in the composition is 100 mass%.

In the present disclosure, more preferably, the content of component (A) is 1-99.9 mass%, and the content of component (B) is 0.1-99 mass%, based on the total amount of components (A) and (B).

In the present disclosure, still more preferably, the content of component (A) is 20-99.5 mass%, and the content of component (B) is 0.5-80 mass%, based on the total amount of components (A) and (B).

In the present disclosure, particularly preferably, the content of component (A) is 50-99 mass%, and the content of component (B) is 1-50 mass%, based on the total amount of components (A) and (B).

As described in Comparative Example 1 later, from the standpoint of dissolving HFE-356mmz in a fluorine oil, the present composition contains no HFIP.

In the present disclosure, the total amount of components (A) and (B) in the total amount of the composition taken as 100 mass% is preferably more than 50 mass%, more preferably 80 mass% or more, still more preferably 90 mass% or more, yet more preferably 99 mass% or more, and particularly preferably 99.5 mass% or more.

It is the most preferable that the present composition consists of components (A) and (B) (with the proviso that unavoidable impurities other than components (A) and (B) are allowed to be present).

It has been unknown that HFE-356mmz and HFE-356mec each exhibit excellent miscibility with a fluorine oil. However, the inventors of the present disclosure, as a result of trial and error of numerous combinations of HFE and oil, surprisingly found that a composition of HFE-356mmz or HFE-356mec with a fluorine oil does not become turbid even when the content of the fluorine oil is set to such a high concentration as described above and becomes transparent due to the high miscibility of HFE-356mmz or HFE-356mec with the fluorine oil.

In the present specification, "transparent" means that the composition is uniform and not turbid.

The present composition can be suitably used as a coating agent because of the excellent miscibility of HFE-356mmz or HFE-356mec with a fluorine oil and the moderately high boiling point of these mixtures.

To achieve the excellent miscibility, the present composition has a boiling point of preferably 40-100°C, and more preferably 50-90°C. When the present composition having a boiling point within these temperature ranges is used in a coating agent, the composition will exhibit excellent solubility with limited evaporation.

In the present specification, the term "coating agent" refers to a mixture that exists in a liquid form at room temperature and that is applied to an article to form a coating film mainly for the purpose of, for example, protecting or imparting luster to the surface of the article.

Another aspect of the present disclosure is a coating method including applying the present composition.

### Optional Additives

The present composition may contain, in addition to HFE-356mmz or HFE-356mec, at least one member selected from additional solvents other than HFE-356mmz or HFE-356mec, antioxidants, stabilizers, preservatives, and surfactants.

Additional solvents for use can be selected from a wide range of solvents such as alkane compounds, alkene compounds, chloroalkane compounds, chloroalkene compounds, hydrofluoroalkane compounds, fluoroalkene compounds, chlorofluoroalkene compounds, and alcohol compounds, according to the properties to be added. However, hexafluoroisopropanol (HFIP) is excluded as such an additional solvent in the present disclosure. For example, for the purpose of dissolving mineral oil, 1,2-dichloroethylene (chloroalkene compound) or 1,2-dichloro-3,3,3-trifluoropropene (chlorofluoroalkene compound) can be added. Isopropyl alcohol (IPA), 1-butanol, or 2,2,3,3,4,4,5,5,6,6,7,7-dodecafluoro-1-heptanol (alcohol compounds), for example, can also be added. In the present disclosure, additional solvents are preferably an alcohol compound. The alcohol compound is preferably at least one member selected from isopropyl alcohol (IPA), 1-butanol, and 2,2,3,3,4,4,5,5,6,6,7,7-dodecafluoro-1-heptanol, and more preferably isopropyl alcohol (IPA). If an additional solvent is added, the present composition contains such an additional solvent in an amount of preferably 0.1 to less than 50 mass%, more preferably 0.5-20 mass%, and still more preferably 1-10 mass%, based on 100 mass% of the composition.

The stabilizers become functional as an acid acceptor or an antioxidant by exerting a stabilization effect. Major stabilization effects include the effect of preventing the decomposition of HFE-356mmz or HFE-356mec by capturing radicals generated in a system, and the acid-accepting effect of preventing further decomposition of HFE-356mmz or HFE-356mec caused by an acid by capturing the acid generated in a system. Such stabilizers for use are selected from a wide range of known stabilizers. In particular, from the standpoint of effectively reducing metal corrosion by the composition, the stabilizer for use is preferably at least one stabilizer selected from unsaturated alcohol-based stabilizers, nitro-based stabilizers, amine-based stabilizers, phenol-based stabilizers, and epoxy-based stabilizers.

The unsaturated alcohol-based stabilizer for use can be selected from a wide range of known such stabilizers. For example, the unsaturated alcohol-based stabilizer for use is at least one member selected from 3-buten-2-ol, 2-buten-1-ol, 4-propen-1-ol, 1-propen-3-ol, 2-methyl-3-buten-2-ol, 3-methyl-3-buten-2-ol, 3-methyl-2-buten-1-ol, 2-hexen-1-ol, 2,4-hexadien-1-ol, and oleyl alcohol.

The nitro-based stabilizer for use can be selected from a wide range of known such stabilizers. Examples of aliphatic nitro compounds include nitromethane, nitroethane, 1-nitropropane, and 2-nitropropane. For example, the aromatic nitro compound for use is at least one member selected from nitrobenzene, o-, m-, or p-dinitrobenzene, o-, m-, or p-nitrotoluene, dimethyl nitrobenzene, m-nitroacetophenone, o-, m-, or p-nitrophenol, o-nitroanisole, m-nitroanisole, and p-nitroanisole.

The amine-based stabilizer for use can be selected from a wide range of known such stabilizers. For example, the amine-based stabilizer for use is at least one member selected from pentylamine, hexylamine, diisopropylamine, diisobutylamine, di-n-propylamine, diallylamine, triethylamine, N-methylaniline, pyridine, morpholine, N-methylmorpholine, triallylamine, allylamine, α-methylbenzylamine, dimethylamine, trimethylamine, ethylamine, diethylamine, triethylamine, propylamine, isopropylamine, dipropylamine, tripropylamine, butylamine, isobutylamine, dibutylamine, tributylamine, dibenzylamine, tribenzylamine, 2-ethylhexylamine, aniline, N,N-dimethylaniline, N,N-diethylaniline, ethylenediamine, propylenediamine, diethylenetriamine, tetraethylenepentamine, benzylamine, dibenzylamine, diphenylamine, and diethylhydroxylamine.

The phenol-based stabilizer for use can be selected from a wide range of known such stabilizers. For example, the phenol-based stabilizer for use is at least one member selected from 2,6-ditertiarybutyl-4-methylphenol, 3-cresol, phenol, 1,2-benzenediol, 2-isopropyl-5-methylphenol, and 2-methoxyphenol.

The epoxy-based stabilizer for use can be selected from a wide range of known such stabilizers. For example, the epoxy-based stabilizer for use is at least one member selected from butylene oxide, 1,2-propylene oxide, 1,2-butylene oxide, butyl glycidyl ether, diethylene glycol diglycidyl ether, and 1,2-epoxy-3-phenoxy propane.

From the standpoint of more effectively preventing the decomposition of HFE-356mmz or HFE-356mec caused by various factors by using a combination of stabilizers having different stabilization effects, the stabilizer is preferably composed of the above-described epoxy-based stabilizer and at least one member selected from the unsaturated alcohol-based stabilizers, nitro-based stabilizers, and phenol-based stabilizers.

From the standpoint of more effectively limiting acid liberation from HFE-356mmz or HFE-356mec and reducing metal corrosion caused by the composition in a liquid form, the content of the stabilizer in the composition taken as 100 mass% is preferably 0.0001 mass% or more, and more preferably 0.01 mass% or more. However, in consideration of avoiding unfavorable changes in physical properties of the composition in a liquid form due to an excessively added stabilizer, the content of the stabilizer in the composition taken as 100 mass% is preferably 10 mass% or less, and more preferably 5 mass% or less.

Another aspect of the present disclosure is a cleaning method including removing a fluorine oil by using a cleaning agent containing HFE-356mmz and/or HFE-356mec and not containing HFIP.

Another aspect of the present disclosure is a dissolution method including dissolving a fluorine oil by using a solvent containing HFE-356mmz and/or HFE-356mec and not containing HFIP.

In these aspects, the fluorine oil to be removed or dissolved is at least one compound selected from the fluorine oils (B) as defined for the present composition above. Of these, perfluoropolyether compounds are preferable.

A composition containing HFE-356mmz and/or HFE-356mec (100 parts by mass) mixed with preferably 20 parts by mass or less of a fluorine oil is usable as a solvent or cleaning agent. Additionally, for example, after the composition is used as a solvent or cleaning agent, the composition containing HFE-356mmz and/or HFE-356mec that remains mixed with a fluorine oil can be used as a solvent or cleaning agent again.

### Examples

Embodiments of the present disclosure are described in more detail with reference to the following Examples.

The following fluorine oils were used in the Examples and Comparative Examples.

· Fluorine Oil (A1): Demnum S-20, produced by Daikin Industries, Ltd., a perfluoropolyether compound (kinematic viscosity at 20°C = 53 cst)
· Fluorine Oil (A2): Demnum S-65, produced by Daikin Industries, Ltd., a perfluoropolyether compound (kinematic viscosity at 20°C = 150 cst)
· Fluorine Oil (A3): Demnum S-200, produced by Daikin Industries, Ltd., a perfluoropolyether compound (kinematic viscosity at 20°C = 500 cst)
· Fluorine Oil (A4): BARRIERTA J100, produced by NOK Klüber Co., Ltd, a perfluoropolyether compound (kinematic viscosity at 20°C = 280 cst)
· Fluorine Oil (A5): Fomblin M30, produced by Solvay Specialty Polymers Japan K.K., a perfluoropolyether compound (kinematic viscosity at 20°C = 280 cst)
· Fluorine Oil (A6): Fomblin Y140/13, produced by Solvay Specialty Polymers Japan K.K., a perfluoropolyether compound (kinematic viscosity at 20°C = 1400 cst)
· Fluorine Oil (A7): OPTOOL UD500, produced by Daikin Industries, Ltd., a silane compound containing a perfluoropolyether group
· Fluorine Oil (A8): DAIFLOIL #20, produced by Daikin Industries, Ltd., a low-molecular polymer of chlorotrifluoroethylene (kinematic viscosity at 25°C = 350 to 1500 cst)
· Fluorine Oil (A9): HOOCCF₂-O-[(CF₂CF₂O)₂₈-(CF₂O)₂₂]-CF₂COOH, produced by Daikin Industries, Ltd., a dicarboxylic acid compound containing a perfluoropolyether group
· Fluorine Oil (A10) : CF₃-O-[(CF₂CF₂O)₂₈-(CF₂O)₂₂]-CF₂COOH, produced by Daikin Industries, Ltd., a monocarboxylic acid compound containing a perfluoropolyether group

### Miscibility Test

### Example 1

1,1,1,3,3,3-Hexafluoroisopropyl methyl ether (HFE-356mmz) and fluorine oil (A1) were placed in a glass test tube to prepare a composition. Whether HFE-356mmz and fluorine oil (A1) were dissolved into each other at 25°C was observed. The content of each component in the composition was HFE-356mmz: 50 mass%, fluorine oil (A1): 50 mass%. The glass test tube was observed, and the composition of HFE-356mmz and fluorine oil (A1) was found to be a single layer and stay in a transparent liquid form. Thus, fluorine oil (A1) was confirmed to have been completely dissolved in HFE-356mmz.

### Example 2

A composition was prepared under the same conditions as in Example 1, except that the content of HFE-356mmz was 20 mass%, and the content of fluorine oil (A1) was 80 mass%. Whether HFE-356mmz and fluorine oil (A1) were dissolved into each other at 25°C was observed. The glass test tube was observed, and the composition of HFE-356mmz and fluorine oil (A1) was found to be a single layer and stay in a transparent liquid form. Thus, fluorine oil (A1) was confirmed to have been completely dissolved in HFE-356mmz.

### Example 3

A composition was prepared under the same conditions as in Example 1, except that the content of HFE-356mmz was 80 mass%, and the content of fluorine oil (A1) was 20 mass%. Whether HFE-356mmz and fluorine oil (A1) were dissolved into each other at 25°C was observed. The glass test tube was observed, and the composition of HFE-356mmz and fluorine oil (A1) was found to be a single layer and stay in a transparent liquid form. Thus, fluorine oil (A1) was confirmed to have been completely dissolved in HFE-356mmz.

### Example 4

A composition was prepared under the same conditions as in Example 1, except that fluorine oil (A2) was used instead of fluorine oil (A1). Whether HFE-356mmz and fluorine oil (A2) were dissolved into each other at 25°C was observed. The glass test tube was observed, and the composition of HFE-356mmz and fluorine oil (A2) was found to be a single layer and stay in a transparent liquid form. Thus, fluorine oil (A2) was confirmed to have been completely dissolved in HFE-356mmz.

### Example 5

A composition was prepared under the same conditions as in Example 1, except that fluorine oil (A3) was used instead of fluorine oil (A1). Whether HFE-356mmz and fluorine oil (A3) were dissolved into each other at 25°C was observed. The glass test tube was observed, and the composition of HFE-356mmz and fluorine oil (A3) was found to be a single layer and stay in a transparent liquid form. Thus, fluorine oil (A3) was confirmed to have been completely dissolved in HFE-356mmz.

### Example 6

A composition was prepared under the same conditions as in Example 1, except that fluorine oil (A4) was used instead of fluorine oil (A1). Whether HFE-356mmz and fluorine oil (A4) were dissolved into each other at 25°C was observed. The glass test tube was observed, and the composition of HFE-356mmz and fluorine oil (A4) was found to be a single layer and stay in a transparent liquid form. Thus, fluorine oil (A4) was confirmed to have been completely dissolved in HFE-356mmz.

### Example 7

A composition was prepared under the same conditions as in Example 1, except that fluorine oil (A5) was used instead of fluorine oil (A1). Whether HFE-356mmz and fluorine oil (A5) were dissolved into each other at 25°C was observed. The glass test tube was observed, and the composition of HFE-356mmz and fluorine oil (A5) was found to be a single layer and stay in a transparent liquid form. Thus, fluorine oil (A5) was confirmed to have been completely dissolved in HFE-356mmz.

### Example 8

A composition was prepared under the same conditions as in Example 1, except that fluorine oil (A6) was used instead of fluorine oil (A1). Whether HFE-356mmz and fluorine oil (A6) were dissolved into each other at 25°C was observed. The glass test tube was observed, and the composition of HFE-356mmz and fluorine oil (A6) was found to be a single layer and stay in a transparent liquid form. Thus, fluorine oil (A6) was confirmed to have been completely dissolved in HFE-356mmz.

### Example 9

A composition was prepared under the same conditions as in Example 1, except that fluorine oil (A7) was used instead of fluorine oil (A1). Whether HFE-356mmz and fluorine oil (A7) were dissolved into each other at 25°C was observed. The glass test tube was observed, and the composition of HFE-356mmz and fluorine oil (A7) was found to be a single layer and stay in a transparent liquid form. Thus, fluorine oil (A7) was confirmed to have been completely dissolved in HFE-356mmz.

### Example 10

A composition was prepared under the same conditions as in Example 1, except that fluorine oil (A8) was used instead of fluorine oil (A1). Whether HFE-356mmz and fluorine oil (A8) were dissolved into each other at 25°C was observed. The glass test tube was observed, and the composition of HFE-356mmz and fluorine oil (A8) was found to be a single layer and stay in a transparent liquid form. Thus, fluorine oil (A8) was confirmed to have been completely dissolved in HFE-356mmz.

### Example 11

A composition was prepared under the same conditions as in Example 1, except that fluorine oil (A9) was used instead of fluorine oil (A1). Whether HFE-356mmz and fluorine oil (A9) were dissolved into each other at 25°C was observed. The glass test tube was observed, and the composition of HFE-356mmz and fluorine oil (A9) was found to be a single layer and stay in a transparent liquid form. Thus, fluorine oil (A9) was confirmed to have been completely dissolved in HFE-356mmz.

### Example 12

A composition was prepared under the same conditions as in Example 1, except that fluorine oil (A10) was used instead of fluorine oil (A1). Whether HFE-356mmz and fluorine oil (A10) were dissolved into each other at 25°C was observed. The glass test tube was observed, and the composition of HFE-356mmz and fluorine oil (A10) was found to be a single layer and stay in a transparent liquid form. Thus, fluorine oil (A10) was confirmed to have been completely dissolved in HFE-356mmz.

### Example 13

1,1,2,3,3,3-Hexafluoropropyl methyl ether (HFE-356mec) and fluorine oil (A1) were placed in a glass test tube to prepare a composition. Whether HFE-356mec and fluorine oil were dissolved into each other at 25°C was observed. The content of each component in the composition was HFE-356mec: 50 mass%, fluorine oil (A1): 50 mass%. The glass test tube was observed, and the composition of HFE-356mec and fluorine oil (A1) was found to be a single layer and stay in a transparent liquid form. Thus, fluorine oil (A1) was confirmed to have been completely dissolved in HFE-356mec.

### Example 14

A composition was prepared under the same conditions as in Example 13, except that fluorine oil (A2) was used instead of fluorine oil (A1). Whether HFE-356mec and fluorine oil (A2) were dissolved into each other at 25°C was observed. The glass test tube was observed, and the composition of HFE-356mec and fluorine oil (A2) was found to be a single layer and stay in a transparent liquid form. Thus, fluorine oil (A2) was confirmed to have been completely dissolved in HFE-356mec.

### Example 15

A composition was prepared under the same conditions as in Example 13, except that fluorine oil (A4) was used instead of fluorine oil (A1). Whether HFE-356mec and fluorine oil (A4) were dissolved into each other at 25°C was observed. The glass test tube was observed, and the composition of HFE-356mec and fluorine oil (A4) was found to be a single layer and stay in a transparent liquid form. Thus, fluorine oil (A4) was confirmed to have been completely dissolved in HFE-356mec.

### Example 16

A composition was prepared under the same conditions as in Example 13, except that fluorine oil (A5) was used instead of fluorine oil (A1). Whether HFE-356mec and fluorine oil (A5) were dissolved into each other at 25°C was observed. The glass test tube was observed, and the composition of HFE-356mec and fluorine oil (A5) was found to be a single layer and stay in a transparent liquid form. Thus, fluorine oil (A5) was confirmed to have been completely dissolved in HFE-356mec.

### Example 17

HFE-356mec and fluorine oil (A6) were placed in a glass test tube to prepare a composition. Whether HFE-356mec and the fluorine oil were dissolved into each other at 25°C was observed. The content of each component in the composition was HFE-356mec: 0.1 mass%, fluorine oil (A6): 99.9 mass%. The glass test tube was observed, and the composition of HFE-356mec and fluorine oil (A6) was found to be a single layer and stay in a transparent liquid form. Thus, fluorine oil (A6) was confirmed to have been completely dissolved in HFE-356mec.

### Example 18

A composition was prepared under the same conditions as in Example 13, except that fluorine oil (A7) was used instead of fluorine oil (A1). Whether HFE-356mec and fluorine oil (A7) were dissolved into each other at 25°C was observed. The glass test tube was observed, and the composition of HFE-356mec and fluorine oil (A7) was found to be a single layer and stay in a transparent liquid form. Thus, fluorine oil (A7) was confirmed to have been completely dissolved in HFE-356mec.

### Example 19

A composition was prepared under the same conditions as in Example 13, except that fluorine oil (A8) was used instead of fluorine oil (A1). Whether HFE-356mec and fluorine oil (A8) were dissolved into each other at 25°C was observed. The glass test tube was observed, and the composition of HFE-356mec and fluorine oil (A8) was found to be a single layer and stay in a transparent liquid form. Thus, fluorine oil (A8) was confirmed to have been completely dissolved in HFE-356mec.

### Example 20

A composition was prepared under the same conditions as in Example 13, except that fluorine oil (A9) was used instead of fluorine oil (A1). Whether HFE-356mec and fluorine oil (A9) were dissolved into each other at 25°C was observed. The glass test tube was observed, and the composition of HFE-356mec and fluorine oil (A9) was found to be a single layer and stay in a transparent liquid form. Thus, fluorine oil (A9) was confirmed to have been completely dissolved in HFE-356mec.

### Example 21

A composition was prepared under the same conditions as in Example 13, except that fluorine oil (A10) was used instead of fluorine oil (A1). Whether HFE-356mec and fluorine oil (A10) were dissolved into each other at 25°C was observed. The glass test tube was observed, and the composition of HFE-356mec and fluorine oil (A10) was found to be a single layer and stay in a transparent liquid form. Thus, fluorine oil (A10) was confirmed to have been completely dissolved in HFE-356mec.

### Example 22

HFE-356mec and isopropyl alcohol (IPA) were placed in a glass test tube to prepare a mixture. The content of each component in the mixture was HFE-356mec: 92 mass%, IPA: 8 mass%. Additionally, fluorine oil (A1) was added to the glass test tube such that the compositional percentages of the mixture and fluorine oil (A1) were respectively 95 mass% and 5 mass%. Whether the mixture and fluorine oil (A1) were dissolved into each other at 25°C was observed. The glass test tube was observed, and the composition of the mixture and fluorine oil (A1) was found to be a single layer and stay in a transparent liquid form. Thus, fluorine oil (A1) was confirmed to have been completely dissolved in the mixture.

### Comparative Example 1

HFE-356mmz and hexafluoroisopropanol (HFIP) were placed in a glass test tube to prepare a mixture. The content of each component in the mixture was HFE-356mmz: 50 mass%, HFIP: 50 mass%. Additionally, fluorine oil (A3) was added to the glass test tube such that the compositional percentages of the mixture and fluorine oil (A3) were respectively 50 mass% and 50 mass%. Whether the mixture and fluorine oil (A3) were dissolved into each other at 25°C was observed. The glass test tube was observed, and the mixture and fluorine oil (A3) were confirmed to have not been dissolved into each other, and to have separated into two layers.

### Comparative Example 2

HFE-356mmz and HFIP were placed in a glass test tube to prepare a mixture. The content of each component in the mixture was HFE-356mmz: 90 mass%, HFIP: 10 mass%. Additionally, fluorine oil (A3) was added to the glass test tube such that the compositional percentages of the mixture and fluorine oil (A3) were respectively 50 mass% and 50 mass%. Whether the mixture and fluorine oil (A3) were dissolved into each other at 25°C was observed. The glass test tube was observed, and the mixture and fluorine oil (A3) were confirmed to have not been dissolved into each other, and to have separated into two layers.

The results of Comparative Example 1 and Comparative Example 2 indicate that the mixture of HFE-356mmz and HFIP is not miscible with fluorine oil (A3).

### Comparative Example 3

In this Comparative Example, bis(2,2,2-trifluoroethyl) ether (HFE-356mff2, CF₃CH₂OCH₂CF₃) was used as an HFE compound. HFE-356mff2 is an HFE compound composed of four carbon atoms, six fluorine atoms, four hydrogen atoms, and one oxygen atom, in the same manner as HFE-356mmz and HFE-356mec in the present disclosure.

Bis(2,2,2-trifluoroethyl) ether (HFE-356mff2, CF₃CH₂OCH₂CF₃) and fluorine oil (A2) were placed in a glass test tube to prepare a composition. Whether HFE-356mff2 and fluorine oil (A2) were dissolved into each other at 25°C was observed. The content of each component in the composition was HFE-356mff2: 50 mass%, fluorine oil (A2): 50 mass%. The glass test tube was observed, and HFE-356mff2 and fluorine oil (A2) were confirmed to have not been dissolved into each other, and to have separated into two layers.

The results of Comparative Example 3 indicate that HFE-356mff2 is not miscible with fluorine oil (A2). It was also confirmed that even HFE compounds composed of the same constituent elements with the same number of those elements, as with HFE-356mmz, HFE-356mec, and HFE-356mff2 (i.e., structural isomers), differ as to whether these compounds can dissolve in fluorine oil or not.

### Comparative Example 4

In this Comparative Example, 1,1,2,2-tetrafluoro-1-(2, 2, 2-trifluoroethoxy) ethane (HFE-347pcf, CF₂HCF₂OCH₂CF₃) was used as an HFE compound.

HFE-347pcf and fluorine oil (A2) were placed in a glass test tube to prepare a composition. Whether HFE-347pcf and fluorine oil (A2) were dissolved into each other at 25°C was observed. The content of each component in the composition was HFE-347pcf: 50 mass%, fluorine oil (A2): 50 mass%. The glass test tube was observed, and HFE-347pcf and fluorine oil (A2) were confirmed to have not been dissolved into each other, and to have separated into two layers.

### Coatability Test

### Example 23

HFE-356mmz and fluorine oil (A2) (lubricant) were mixed in a glass test tube to prepare a lubricant solution. The content of each component in the lubricant solution was HFE-356mmz: 97 mass%, fluorine oil (A2): 3 mass%. Subsequently, the lubricant solution was applied to the surface of an aluminum substrate to give an average thickness of 0.5 mm, and dried in air at 24 to 27°C, thereby forming a lubricant coating film on the surface of the aluminum substrate. The lubricant coating film was visually observed and confirmed to be a uniform coating film.

### Stability Test

### Example 24

HFE-356mmz and fluorine oil (A2) were mixed in a glass test tube to prepare a solution. The content of each component in the solution was HFE-356mmz: 50 mass%, fluorine oil (A2): 50 mass%. After the obtained solution was allowed to stand at 25°C for 30 days, the state of the solution was visually observed. The solution was confirmed to have undergone no changes in color, and precipitation formation.

### Cleaning Test

### Example 25

A glass test piece (50 mm × 5 mm × 2 mm) was immersed in fluorine oil (A2) for 30 seconds. The glass test piece was then immersed in 100 ml of HFE-356mmz for 1 minute at 25°C and pulled out, followed by drying in air at room temperature for 5 minutes. As a result, fluorine oil (A2) remaining on the glass test piece was confirmed to have been removed.

### Coatability Test

### Example 26

HFE-356mec and fluorine oil (A2) (lubricant) were mixed in a glass test tube to prepare a lubricant solution. The content of each component in the lubricant solution was HFE-356mec: 97 mass%, fluorine oil (A2): 3 mass%. Subsequently, the lubricant solution was applied to the surface of an aluminum substrate to give an average thickness of 0.5 mm and dried in air at 24 to 27°C, thereby forming a lubricant coating film on the surface of the aluminum substrate. The lubricant coating film was visually observed and confirmed to be a uniform coating film.

### Stability Test

### Example 27

HFE-356mec and fluorine oil (A2) were mixed in a glass test tube to prepare a solution. The content of each component in the solution was HFE-356mmz: 50 mass%, fluorine oil (A2): 50 mass%. After the obtained solution was allowed to stand at 25°C for 30 days, the state of the solution was visually observed. The solution was confirmed to have undergone no changes in color, and precipitation formation.

### Cleaning Test

### Example 28

A glass test piece (50 mm × 5 mm × 2 mm) was immersed in fluorine oil (A2) for 30 seconds. The glass test piece was then immersed in 100 ml of HFE-356mec for 1 minute at 25°C and pulled out, followed by drying in air at room temperature for 5 minutes. As a result, fluorine oil (A2) remaining on the glass test piece was confirmed to have been removed.

## Claims

1. A composition comprising
(A) 1,1,1,3,3,3-hexafluoroisopropyl methyl ether (HFE-356mmz) and/or 1,1,2,3,3,3-hexafluoropropyl methyl ether (HFE-356mec), and
(B) a fluorine oil, which is at least one compound selected from
- a perfluoropolyether compound,
- a monocarboxylic acid compound, a dicarboxylic acid compound or a silane compound, each containing a perfluoropolyether group, and
- a polymer of chlorotrifluoroethylene having a number average molecular weight of ≤ 10,000,
and which composition does not contain hexafluoroisopropanol (HFIP).

2. The composition of claim 1, which contains 1-99.99 mass% of component (A) and 0.01-99 mass% of component (B), based on the total amount of components (A) and (B).

3. The use of the composition of claim 1 or 2 in a coating agent which is a mixture existing in liquid form at room temperature and is applied to an article to form a coating film for protecting or imparting luster to the surface of the article.

4. A cleaning method comprising removing a fluorine oil (B) as defined in claim 1, using a cleaning agent which contains HFE-356mmz and/or HFE-356mec and does not contain HFIP.

5. A dissolution method comprising dissolving a fluorine oil (B) as defined in claim 1, using a solvent which contains HFE-356mmz and/or HFE-356mec and does not contain HFIP.

## Patentansprüche

1. Zusammensetzung, umfassend
(A) 1,1,1,3,3,3-Hexafluorisopropylmethylether (HFE-356mmz) und/oder 1,1,2,3,3,3-Hexafluorpropylmethylether (HFE-356mec), und
(B) ein Fluoröl, das mindestens eine Verbindung ist, ausgewählt aus
- einer Perfluorpolyetherverbindung,
- einer Monocarbonsäureverbindung, einer Dicarbonsäureverbindung oder einer Silanverbindung, die jeweils eine Perfluorpolyethergruppe enthalten, und
- ein Polymer von Chlortrifluorethylen mit einem zahlenmittleren Molekulargewicht von ≤ 10.000,
und wobei die Zusammensetzung kein Hexafluorisopropanol (HFIP) enthält.

2. Zusammensetzung gemäß Anspruch 1, die enthält 1-99,99 Massen-% der Komponente (A) und 0,01-99 Massen-% der Komponente (B), bezogen auf die Gesamtmenge der Komponenten (A) und (B).

3. Verwendung der Zusammensetzung gemäß Anspruch 1 oder 2 in einem Beschichtungsmittel, bei dem es sich um ein Gemisch handelt, das bei Raumtemperatur in flüssiger Form vorliegt und auf einen Gegenstand aufgetragen wird, um einen Beschichtungsfilm zu bilden, zum Schutz oder zur Verleihung von Glanz der Oberfläche des Gegenstands.

4. Reinigungsverfahren, umfassend die Entfernung eines Fluoröls (B) wie in Anspruch 1 definiert, unter Verwendung eines Reinigungsmittels, das HFE-356mmz und/oder HFE-356mec enthält und kein HFIP enthält.

5. Lösungsverfahren, umfassend das Lösen eines Fluoröls (B) gemäß Anspruch 1 unter Verwendung eines Lösungsmittels, das HFE-356mmz und/oder HFE-356mec und kein HFIP enthält.

## Revendications

1. Composition comprenant
(A) de l'éther méthylique de 1,1,1,3,3,3-hexafluoroisopropyle (HFE-356mmz) et/ou de l'éther méthylique de 1,1,2,3,3,3-hexafluoropropyle (HFE-356mec), et
(B) une huile fluorée, qui est au moins un composé sélectionné parmi
- un composé de perfluoropolyéther,
- un composé d'acide monocarboxylique, un composé d'acide dicarboxylique ou un composé de silane, chacun contenant un groupe perfluoropolyéther, et
- un polymère de chlorotrifluoroéthylène présentant un poids moléculaire moyen en nombre de ≤ 10 000,
et laquelle composition ne contient pas d'hexafluoroisopropanol (HFIP).

2. Composition selon la revendication 1, qui contient de 1 à 99,99 % en masse du composant (A) et de 0,01 à 99 % en masse du composant (B), sur la base de la quantité totale des composants (A) et (B).

3. Utilisation de la composition selon la revendication 1 ou 2 dans un agent de revêtement qui est un mélange existant sous forme liquide à température ambiante et est appliqué à un article pour former un film de revêtement destiné à protéger la surface de l'article ou conférer un éclat à cette dernière.

4. Procédé de nettoyage comprenant le retrait d'une huile fluorée (B) telle que définie dans la revendication 1, en utilisant un agent de nettoyage qui contient du HFE-356mmz et/ou du HFE-356mec et ne contient pas de HFIP.

5. Procédé de dissolution comprenant la dissolution d'une huile fluorée (B) telle que définie dans la revendication 1, en utilisant un solvant qui contient du HFE-356mmz et/ou du HFE-356mec et ne contient pas de HFIP.
